# EUROPEAN PATENT SPECIFICATION

(11) **EP 3 510 936 B1**
(45) Date of publication and mention of the grant of the patent: **09.11.2022**
(21) Application number: 18160296.2
(22) Date of filing: 06.03.2018
(51) Int. Cl.: A61B 8/06, A61B 8/00, A61B 8/08, G16H 50/30

(54) **ULTRASOUND IMAGING APPARATUS AND METHOD OF CONTROLLING THE SAME**
ULTRASCHALLBILDGEBUNGSVORRICHTUNG UND VERFAHREN ZUR STEUERUNG DAVON
APPAREIL D'IMAGERIE À ULTRASONS ET PROCÉDÉ DE COMMANDE CORRESPONDANT

(30) Priority: 11.01.2018 KR 20180003974
(43) Date of publication of application: 17.07.2019
(73) Proprietor: Samsung Medison Co., Ltd., Hongcheon-gun, Gangwon-do, 25108 (KR)
(72) Inventor: Kang, Sung-min, 25108 Gangwon-do (KR); Yang, Eun-ho, 25108 Gangwon-do (KR)
(74) Representative: Arnold & Siedsma

(56) References cited:
- WO-A1-2016/172890
- US-A1- 2006 052 698
- US-A1- 2013 150 717

## Description

One or more embodiments relate to an ultrasound imaging apparatus and a method of controlling the same.

Ultrasound imaging apparatuses transmit an ultrasound signal generated by a transducer of a probe to an object and detect information about a signal reflected from the object, thereby obtaining at least one image of an internal part (e.g., soft tissue or blood flow) of the object.

Also, ultrasound imaging apparatuses may measure a speed and a direction of a moving object by using the Doppler effect and may output the measured speed and direction of the object. For example, ultrasound imaging apparatuses may measure a speed and a direction of a moving muscle or blood flow in the heart or a carotid artery.

Document US2006/052698 discloses the generation of spectral Doppler images on the basis of regions-of-interest in an ultrasound image.

One or more embodiments include an ultrasound imaging apparatus for providing a spectral Doppler image of an object to a user, and a method of controlling the ultrasound imaging apparatus.

One or more embodiments include an ultrasound imaging apparatus for providing a plurality of spectral Doppler images respectively corresponding to a plurality of sections of a region of interest (ROI) of an object, and a method of controlling the ultrasound imaging apparatus.

Additional aspects will be set forth in part in the description which follows and, in part, will be apparent from the description, or may be learned by practice of the presented embodiments.

According to one or more embodiments, an ultrasound imaging apparatus according to claim 1 is disclosed.

The at least one processor may be further configured to divide the set ROI into the plurality of sections based on a user input for at least one or a combination of a number of the plurality of sections, a size of each of the plurality of sections, and a shape of each of the plurality of sections.

The at least one processor may be further configured to divide the set ROI into the plurality of sections by automatically recognizing an anatomical structure in the set ROI and automatically determining, based on the recognized anatomical structure, at least one or a combination of a number of the plurality of sections, a size of each of the plurality of sections, and a shape of each of the plurality of sections.

The at least one processor may be further configured to update the determined display order in each of preset cycles.

The at least one processor may be further configured to set a color corresponding to at least one of the plurality of sections, and the display may be further configured to display at least one of the plurality of sections of the first region and at least one of the plurality of spectral Doppler images of the second region by applying the set color to the at least one of the plurality of sections of the first region and the at least one of the plurality of spectral Doppler images of the second region.

The at least one processor may be further configured to classify the plurality of spectral Doppler images into groups having the same cycle, the same phase, and the same pattern of blood flow, based on blood flow data, and the display may be further configured to display the plurality of spectral Doppler images according to the groups on different portions of the second region in the display order.

The at least one processor may be further configured to set a color corresponding to each of the classified groups, and the display may be further configured to apply the set color to at least one of the plurality of sections of the first region and at least one of the plurality of spectral Doppler images of the second region.

With respect to the plurality of spectral Doppler images classified into the groups, the at least one processor may be further configured to synthesize, for each group, spectral Doppler images included in a group into one spectral Doppler image having a continuous spectrum on the same time axis, and the display may be further configured to display the synthesized spectral Doppler images on different portions of the second region according to the groups.

The at least one processor may be further configured to set a color corresponding to each of the classified groups, and the display may be further configured to apply the set color to at least one of the plurality of sections of the first region and at least one of the plurality of spectral Doppler images of the second region.

The at least one processor may be further configured to re-set the ROI so that a section corresponding to a spectral Doppler image having a highest peak velocity value of blood flow from among the plurality of spectral Doppler images is located at the center of the ROI.

According to one or more embodiments, a method of controlling an ultrasound imaging apparatus according to claim 11 is disclosed.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating a configuration of an ultrasound diagnosis apparatus according to an embodiment;
FIGS. 2A, 2B, and 2C are views illustrating ultrasound diagnosis apparatuses according to embodiments;
FIG. 3 is a block diagram illustrating a structure of an ultrasound imaging apparatus, according to an embodiment;
FIG. 4 is a flowchart of a method by which the ultrasound imaging apparatus displays a spectral Doppler image of an object, according to an embodiment;
FIGS. 5A, 5B, and 5C are views illustrating an example where the ultrasound imaging apparatus sets a region of interest (ROI) in an ultrasound image of an object and divides the ROI into a plurality of sections, according to an embodiment;
FIGS. 6A and 6B are views illustrating an example where the ultrasound imaging apparatus displays an ultrasound image of an object and a plurality of spectral Doppler images on a display, according to an embodiment;
FIG. 7 is a view illustrating an example where the ultrasound imaging apparatus displays an ultrasound image of an object and a plurality of spectral Doppler images on the display according to groups having the same cycle and the same pattern of blood flow, according to an embodiment;
FIG. 8 is a view illustrating an example where the ultrasound imaging apparatus displays an ultrasound image of an object and a plurality of spectral Doppler images synthesized according to groups having the same cycle and the same pattern of blood flow on the display, according to an embodiment; and
FIG. 9 is a view illustrating an example where the ultrasound imaging apparatus re-sets an ROI so that a section having a highest peak velocity value of blood flow in an ultrasound image of an object is located at the center of the ROI, according to an embodiment.

### DETAILED DESCRIPTION

Hereinafter, principles and embodiments of the present disclosure will be described in detail in order to fully convey the scope of the present disclosure and enable one of ordinary skill in the art to embody and practice the present disclosure. The embodiments may be implemented in various forms.

The same reference numerals denote the same elements throughout the specification. All elements of embodiments are not described in the specification, and descriptions of matters well known in the art to which the present disclosure pertains or repeated descriptions between embodiments will not be given. Terms such as 'module' and 'unit' used herein denote those that may be embodied by software, hardware, or firmware, or any combination thereof. According to embodiments, a plurality of 'modules' or 'units' may be embodied by a single element, or a single 'module' or 'unit' may include a plurality of elements.

Operation principles and embodiments of the present disclosure will now be explained with reference to the accompanying drawings.

In embodiments, an image may include a medical image acquired by any of various medical imaging apparatuses such as a magnetic resonance imaging (MRI) apparatus, a computed tomography (CT) apparatus, an ultrasound imaging apparatus, or an X-ray apparatus.

Also, in the present specification, an 'object', which is a thing to be imaged, may include a human, an animal, or a part thereof. For example, an object may include a part of a human (e.g., an organ or a tissue) or a phantom.

Throughout the specification, an "ultrasound image" refers to an image of an object processed based on ultrasound signals transmitted to the object and reflected therefrom.

Embodiments will now be described more fully with reference to the accompanying drawings.

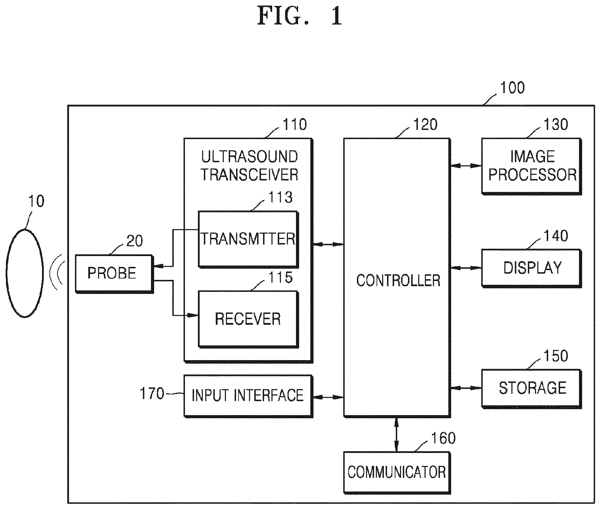

FIG. 1 is a block diagram illustrating a configuration of an ultrasound diagnosis apparatus 100 according to an embodiment. The ultrasound diagnosis apparatus 100 according to an embodiment may include a probe 20, an ultrasound transceiver 110, a controller 120, an image processor 130, a display 140, a storage 150, a communicator 160, and an input interface 170.

The ultrasound diagnosis apparatus 100 may be of a cart-type or portable-type ultrasound diagnosis apparatus. Examples of the portable-type ultrasound diagnosis apparatus 100 may include, but not limited to, a smart phone, a laptop computer, a personal digital assistant (PDA), and a tablet personal computer (PC).

The probe 20 may include a plurality of transducers. The plurality of transducers may transmit ultrasound signals to an object 10 in response to transmission signals applied from a transmitter 113. The plurality of transducers may receive ultrasound signals reflected from the object 10 to generate reception signals. In addition, the probe 20 and the ultrasound diagnosis apparatus 100 may be formed in one body, or the probe 20 and the ultrasound diagnosis apparatus 100 may be formed separately but linked wirelessly or via wires. In addition, the ultrasound diagnosis apparatus 100 may include one or more probes 20 according to embodiments.

The controller 120 may control the transmitter 113 to generate transmission signals to be applied to the plurality of transducers included in the probe 20 in consideration of a position and a focal point of each of the plurality of transducers.

The controller 120 may control a receiver 115 to generate ultrasound data by converting reception signals received from the probe 20 from analogue to digital signals and summing the reception signals converted into digital form in consideration of the position and the focal point of each of the plurality of transducers.

The image processor 130 may generate an ultrasound image by using the ultrasound data generated by the receiver 115.

The display 140 may display the generated ultrasound image and various pieces of information processed by the ultrasound diagnosis apparatus 100. The ultrasound diagnosis apparatus 100 may include one or more displays 140 according to embodiments. The display 140 may include a touch screen in combination with a touch panel.

The controller 120 may control operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses (e.g., servers, medical apparatuses, and portable devices such as smart phones, tablet personal computers (PCs), or wearable devices) via the communicator 160.

The communicator 160 may include at least one element capable of communicating with the external apparatuses. For example, the communicator 160 may include at least one from among a short-range communication module, a wired communication module, and a wireless communication module.

The communicator 160 may transmit and receive a control signal and data to and from the external apparatuses.

The storage 150 may store various data or programs for driving and controlling the ultrasound diagnosis apparatus 100, input and/or output ultrasound data, obtained ultrasound images, etc.

The input interface 170 may receive a user's input for controlling the ultrasound diagnosis apparatus 100. For example, the user's input may include, but not limited to, inputs for manipulating buttons, keypads, mice, trackballs, jog switches, or knops, inputs for touching a touchpad or a touch screen, a voice input, a motion input, and a bio-information input (e.g., iris recognition or fingerprint recognition).

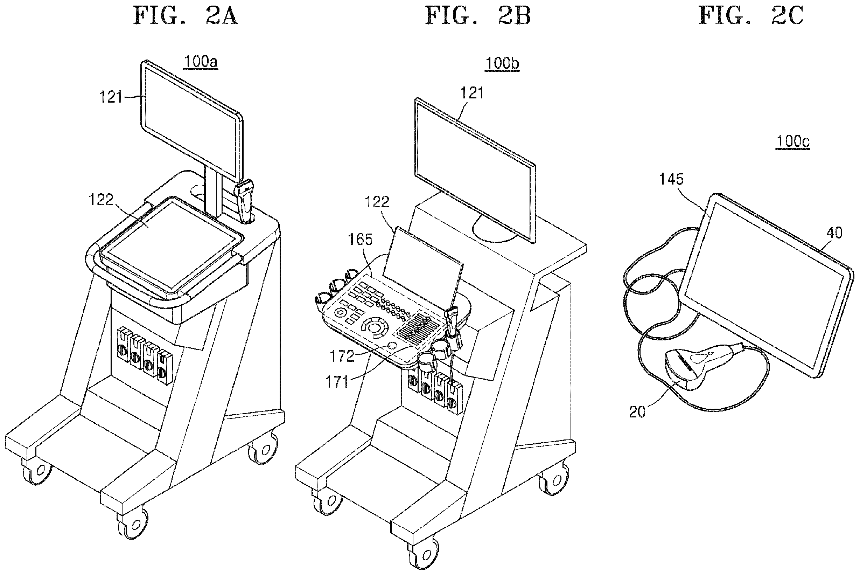

The ultrasound diagnosis apparatus 100 according to an embodiment will be described with reference to FIGS. 2A, 2B, and 2C.

FIGS. 2A, 2B, and 2C are views illustrating ultrasound diagnosis apparatuses 100a, 100b, and 100c according to embodiments.

Referring to FIGS. 2A and 2B, each of the ultrasound diagnosis apparatuses 100a and 100b may include a main display 121 and a sub-display 122. One of the main display 121 and the sub-display 122 may include a touch screen. The main display 121 and the sub-display 122 may display ultrasound images and/or various information processed by each of the ultrasound diagnosis apparatuses 100a and 100b. The main display 121 and the sub-display 122 may provide graphical user interfaces (GUI), thereby receiving data for controlling each of the ultrasound diagnosis apparatuses 100a and 100b from a user. For example, the main display 121 may display an ultrasound image and the sub-display 122 may display a control panel for controlling display of the ultrasound image as a GUI. The sub-display 122 may receive data for controlling display of an image through the control panel displayed as a GUI. Each of the ultrasound diagnosis apparatuses 100a and 100b may control the display of the ultrasound image on the main display 121 by using the received control data.

Referring to FIG. 2B, the ultrasound diagnosis apparatus 100b may further include a control panel 165 in addition to the main display 121 and the sub-display 122. The control panel 165 may include buttons, trackballs, jog switches, or knops, and may receive data for controlling the ultrasound diagnosis apparatus 100b from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100b may keep displaying a frame image at that time point.

The buttons, trackballs, jog switches, and knops included in the control panel 165 may be provided as a GUI to the main display 121 or the sub-display 122.

Referring to FIG. 2C, the ultrasound diagnosis apparatus 100c may be a portable device. Examples of the portable ultrasound diagnosis apparatus 100 may include, but not limited to, smart phones including probes and applications, laptop computers, PDAs, and tablet PCs.

The ultrasound diagnosis apparatus 100c may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100c, and a GUI.

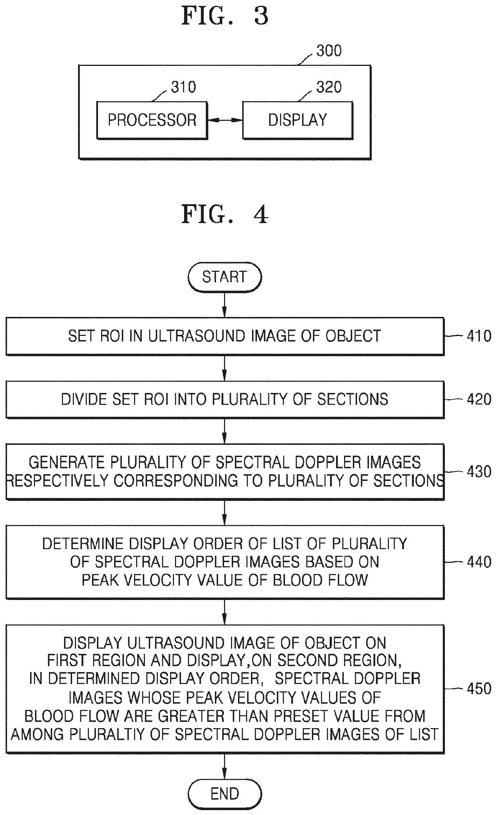

FIG. 3 is a block diagram illustrating a structure of an ultrasound imaging apparatus 300, according to an embodiment. The ultrasound imaging apparatus 300 may correspond to the ultrasound diagnosis apparatus 100 of FIG. 1. Also, the ultrasound imaging apparatus 300 may be implemented as any of the ultrasound diagnosis apparatuses 100a, 100b, and 100c of FIGS. 2A through 2C.

As shown in FIG. 3, the ultrasound imaging apparatus 300 includes a processor 310 and a display 320. The processor 310 may correspond to the controller 120 and the image processor 130 of FIG. 1. The display 320 may correspond to the display 140 of FIG. 1. Also, the processor 310 may include one or more processors.

The processor 310 may set a region of interest (ROI) in an ultrasound image of an object. According to an embodiment, the ROI in the ultrasound image of the object may be set based on a user input. For example, when a user designates two points on an ultrasound image screen, a rectangular ROI with a diagonal that connects the two points may be set.

The processor 310 may divide the set ROI into a plurality of sections. In an embodiment, the dividing of the set ROI into the plurality of sections may be performed based on the user's input for at least one or a combination of the number of the plurality of sections, a size of each of the plurality of sections, and a shape of each of the plurality of sections.

For example, the ultrasound imaging apparatus 300 may receive a user input that sets the number of a plurality of sections divided from a rectangular ROI to N, a shape of each of the plurality of sections to a rectangular shape, and a size of each of the plurality of sections to 1/N of a size of the rectangular ROI. The processor 310 may divide the ROI set based on the received user input into N sections having rectangular shapes and the same size.

According to another embodiment, the dividing of the set ROI into the plurality of sections may be automatically performed. The processor 310 may automatically recognize an anatomical structure in the set ROI. The processor 310 may divide the set ROI into the plurality of sections by automatically determining at least one or a combination of the number of the plurality of sections, a size of each of the plurality of sections, and a shape of each of the plurality of sections based on the recognized anatomical structure.

For example, when the object is set to a fetus and the ROI is set to a region including fetal umbilical vessels made of twisted arteries and veins, the ROI may be divided as follows. The processor 310 may automatically recognize the fetal umbilical vessels in the set ROI. The processor 310 may divide the set ROI into a plurality of sections by automatically determining at least one or a combination of the number of the plurality of sections, a size of each of the plurality of sections, and a shape of each of the plurality of sections based on shapes and sizes of the recognized fetal umbilical vessels. For example, the processor 310 may define the plurality sections so that a region corresponding to each artery of the umbilical vessels and a region corresponding to each vein of the umbilical vessels are divided as different sections.

Each of the plurality of sections of the ROI is not limited to a rectangular shape, and may have any of various other shapes such as a triangular shape, a quadrangular shape, a pentagonal shape, or a hexagonal shape. Also, shapes of the plurality of sections of the ROI may be different from one another. Sizes of the plurality of sections of the ROI may be different from one another.

The processor 310 may obtain blood flow data corresponding to each of the plurality of sections. The blood flow data may include at least one or a combination of a velocity, a cycle, and a pattern of blood flow.

The velocity of blood flow includes data about at least one or a combination of a magnitude of a velocity of blood flow with time, a peak velocity value of blood flow within one cycle, and an average velocity value of blood flow during one cycle. The cycle of blood flow refers to a predetermined time interval during which a level of blood flow is repeatedly changed. The pattern of blood flow that refers to changes in a level of blood flow with time during one cycle may include a waveform, a phase, and a cycle of blood flow with time.

The processor 310 may generate a plurality of spectral Doppler images respectively corresponding to the plurality of sections of the ROI based on the obtained blood flow data. The spectral Doppler images refer to images in which a velocity of blood flow with time is represented as a waveform.

The processor 310 may determine a display order of a list of the generated plurality of spectral Doppler images based on the velocity of blood flow.

According to an embodiment, the processor 310 may determine the display order of the list of the plurality of spectral Doppler images based on the peak velocity value of blood flow. The processor 310 may compare peak velocity values of blood flow corresponding to the plurality of spectral Doppler images based on the blood flow data and may determine a magnitude order of the peak velocity values of blood flow as the display order of the list of the plurality of spectral Doppler images. Each peak velocity value of blood flow may refer to the absolute value of a magnitude of a peak velocity of blood flow.

The processor 310 may update the display order of the plurality of spectral Doppler images in every preset cycle. According to an embodiment, the processor 310 may set an update cycle of the display order based on the user's input for the update cycle of the display order.

When the object is moving, portions of the object corresponding to the plurality of sections may be changed. Once the portions of the object corresponding to the plurality of sections are changed, magnitudes of peak velocity values of blood flow of the plurality of spectral Doppler images respectively corresponding to the plurality of sections may also be changed. Accordingly, the plurality of spectral Doppler images may be displayed in the magnitude order of the peak velocity values of blood flow in real time by updating the display order in every preset cycle.

The processor 310 may generate a list of the plurality of spectral Doppler images based on the display order of the plurality of spectral Doppler images. According to an embodiment, the plurality of spectral Doppler images may be located from the top to the bottom of the list in the magnitude order of the peak velocity values of blood flow according to the display order of the plurality of spectral Doppler images.

The display 320 may display the ultrasound image of the object on a first region, and the plurality of spectral Doppler images on a second region.

According to an embodiment, the display 320 may display the ultrasound image of the object on the first region in at least one of a B mode, a C mode, and a PW mode, or a combination thereof.

According to an embodiment, the display 320 may display a plurality of spectral Doppler images in the list of the plurality of Doppler images on the second region in the display order determined by the processor 310. For example, the display 320 may display the plurality of spectral Doppler images arranged in the magnitude order of the peak velocity values of blood flow in the list, on the second region from the top to the bottom of the second region.

According to an embodiment, since the ultrasound imaging apparatus 300 displays the plurality of spectral Doppler images in the magnitude order of the peak velocity values of blood flow, the user may easily find a portion having a highest velocity of blood flow in the ROI.

For example, according to an embodiment, even when a position where cardiac regurgitation occurs is periodically changed, the user may easily find a position where cardiac regurgitation occurs and may diagnose the cardiac regurgitation. In detail, the ultrasound imaging apparatus 300 may set the heart as an ROI and may divide the set ROI into a plurality of sections. The ultrasound imaging apparatus 300 may display spectral Doppler images respectively corresponding to the plurality of sections based on a velocity of blood flow. A spectral Doppler image displayed at the top may be a spectral Doppler image at a position where cardiac regurgitation occurs. Accordingly, the user may easily diagnose the cardiac regurgitation by using the spectral Doppler image displayed at the top.

According to an embodiment, the display 320 may display, on the second region, in the display order determined by the processor 310, spectral Doppler images whose peak velocity values of blood flow are greater than a preset value from among the plurality of spectral Doppler images in the list of the plurality of spectral Doppler images. The preset value may be an initial set value when there is no additional user input, and may be re-set by the user's input.

The probability that a section corresponding to a spectral Doppler image whose peak velocity value of blood flow is less than a predetermined value does not include a blood vessel is high. Accordingly, a spectral Doppler image whose peak velocity value of blood flow is less than a predetermined value may not be a spectral Doppler image of a portion that is desired by the user to be diagnosed. Accordingly, the efficiency of diagnosis of a portion of the object desired by the user to be diagnosed may be improved by selectively displaying only spectral Doppler images whose peak velocity values of blood flow are greater than a predetermined value.

FIG. 4 is a flowchart of a method by which the ultrasound imaging apparatus 300 displays a spectral Doppler image of an object, according to an embodiment.

Referring to FIG. 4, a method of displaying a spectral Doppler image of an object according to another embodiment includes operations sequentially performed by the ultrasound imaging apparatus 300 of FIG. 3. Accordingly, although omitted, the description of the ultrasound imaging apparatus 300 of FIG. 3 may apply to the method of displaying the spectral Doppler image of the object of FIG. 4.

In operation 410, the ultrasound imaging apparatus 300 sets an ROI in an ultrasound image of an object. The setting of the ROI may be performed based on a user's input for a size and a shape of the ROI.

In operation 420, the ultrasound imaging apparatus 300 divides the set ROI into a plurality of sections. The ultrasound imaging apparatus 300 may divide the ROI into the plurality of sections based on the user's input, and may automatically divide the ROI into the plurality of sections by analyzing an anatomical structure in the ROI.

In operation 430, the ultrasound imaging apparatus 300 generates a plurality of spectral Doppler images respectively corresponding to the plurality of sections. The spectral Doppler images are images in which a velocity of blood flow with time is represented as a waveform.

In operation 440, the ultrasound imaging apparatus 300 determines a display order of a list of the plurality of spectral Doppler images based on a peak velocity value of blood flow.

In operation 450, the ultrasound imaging apparatus 300 displays the ultrasound image of the object on a first region of the display 320, and displays, on a second region, in the determined display order, spectral Doppler images whose peak velocity values of blood flow are greater than a preset value from among the plurality of spectral Doppler images of the list.

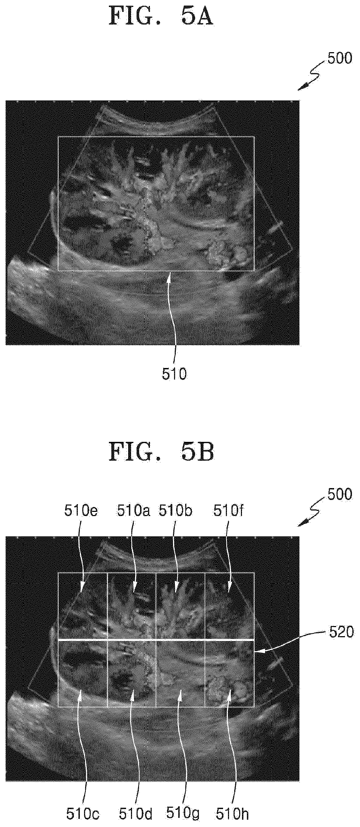

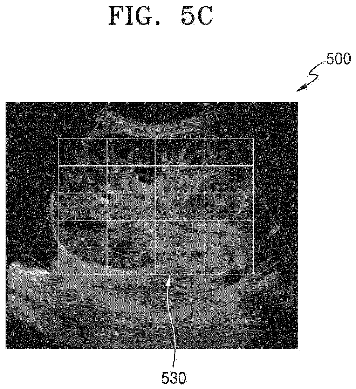

FIGS. 5A, 5B, and 5C are views illustrating an example where the ultrasound imaging apparatus 300 sets an ROI in an ultrasound image of an object and divides the ROI into a plurality of sections, according to an embodiment.

FIG. 5A illustrates an example where the ultrasound imaging apparatus 300 sets an ROI 510 to a rectangular shape in an ultrasound image 500 of a kidney of an object. Referring to FIG. 5A, the ROI 510 is set to include a blood vessel of the kidney.

FIG. 5B illustrates a divided ROI 520 obtained when the ultrasound imaging apparatus 300 divides the ROI 510 set in the ultrasound image 500 of the kidney of the object into 8 sections having the same size.

According to an embodiment, the ultrasound imaging apparatus 300 may obtain the divided ROI 520 of FIG. 5B based on a user's input commanding that the ROI 510 be divided into 8 sections having the same size.

According to another embodiment, the ultrasound imaging apparatus 300 may divide the ROI 510 by automatically recognizing a renal vascular structure in the ROI 510. The ultrasound imaging apparatus 300 may divide the ROI 510 into a plurality of sections 510a, 510b, 510c, ..., and 510h of the divided ROI 520 of FIG. 5B by determining the number of the plurality of sections to be 8 and determining a size of each of the plurality of sections to be 1/8 of a size of the ROI 510 based on the recognized renal vascular structure.

As shown in FIG. 5B, some sections (e.g., 510a, 510b, 510c, and 510d) in the divided ROI 520 include blood vessels of the kidney. Accordingly, the user may efficiently diagnose fine renal blood vessels by using spectral Doppler images of the some sections (e.g., 510a, 510b, 510c, and 510d) including the blood vessels of the kidney provided by the ultrasound imaging apparatus 300.

FIG. 5C illustrates an ROI 530 obtained when the ultrasound imaging apparatus 300 divides the ROI 510 set in the ultrasound image 500 of the kidney of the object into 20 sections having the same size.

It is difficult for the user to diagnose blood vessels of an object having a complex anatomical structure such as a kidney including fine blood vessels because it is more difficult to measure blood flow of the object having the complex anatomical structure than to measure blood flow of an object having a simple anatomical structure. However, according to an embodiment, a desired image of even a blood vessel having a fine structure may be obtained by dividing an ROI into a larger number of sections and generating spectral Doppler images of the divided sections.

Also, according to an embodiment, even when an object is moving, the ultrasound imaging apparatus 300 may generate spectral Doppler images of a plurality of sections of an ROI in real time. Accordingly, although sections in an ROI corresponding to portions desired by the user to be diagnosed may be changed after the movement of the object, spectral Doppler images of the sections in the ROI corresponding to the portions desired by the user to be diagnosed may be obtained even after the movement of the object, and thus seamless spectral Doppler images may be obtained.

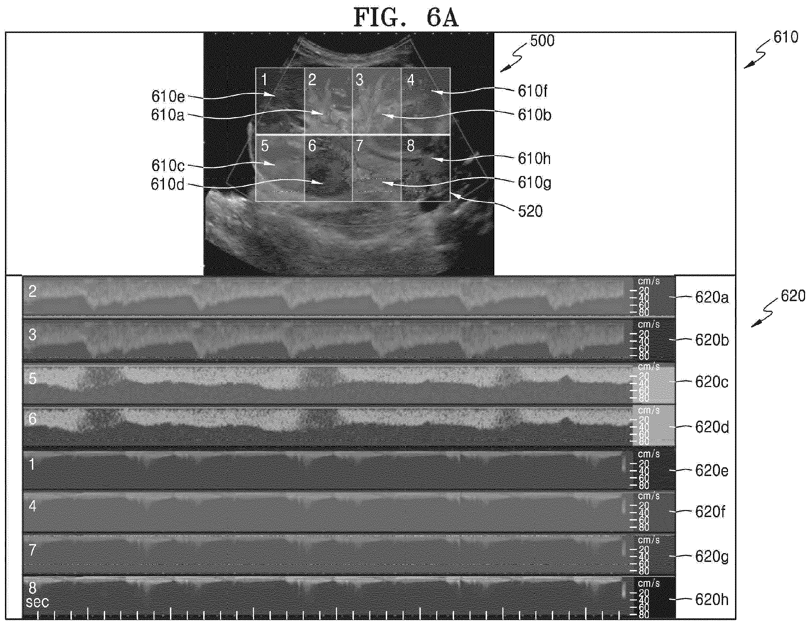

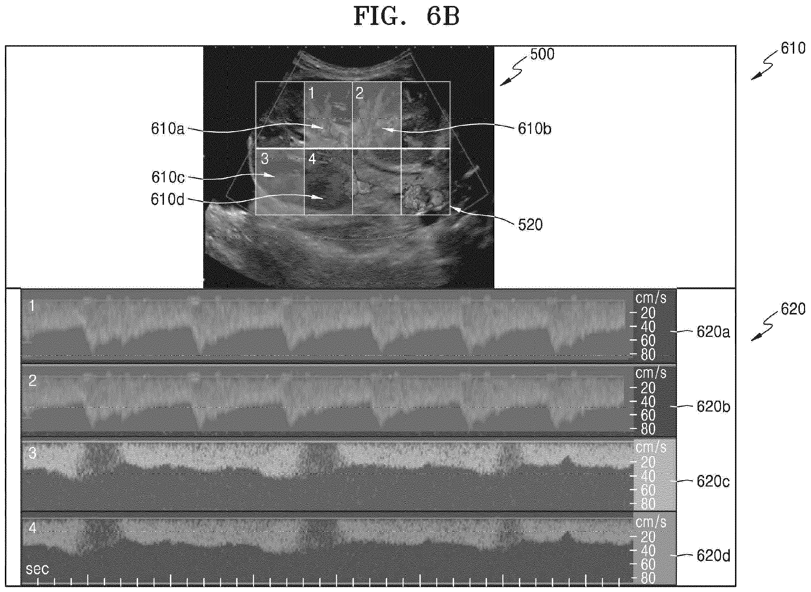

FIGS. 6A and 6B are views illustrating an example where the ultrasound imaging apparatus 300 displays an ultrasound image 500 of an object on a first region 610 of the display 320 and displays a plurality of spectral Doppler images on a second region 620, according to an embodiment.

According to an embodiment, as shown in FIGS. 6A and 6B, the display 320 may vertically locate the first region 610 where an ultrasound image of an object is displayed and the second region 620 where a plurality of spectral Doppler images are displayed.

According to another embodiment, the display 320 may horizontally locate a first region where an ultrasound image of an object is displayed and a second region where a plurality of spectral Doppler images are displayed.

According to another embodiment, the display 320 may change positions of a first region where an ultrasound image of an object is displayed and a second region where a plurality of Doppler images are displayed, based on a user's input.

FIG. 6A is a view illustrating an example where the ultrasound image 500 of an object is displayed on the first region 610 of the display 320, and a plurality of spectral Doppler images respectively corresponding to a plurality of sections of the divided ROI 520 of FIG. 5B are displayed on the second region 620 of the display 320. According to an embodiment, the processor 310 may determine a display order of a list of the plurality of spectral Doppler images based on a peak velocity value of blood flow. The display 320 may display a plurality of spectral Doppler images of the list on the second region 620 in the determined display order.

Referring to FIG. 6A, the display 320 displays a plurality of spectral Doppler images respectively corresponding to 8 sections (e.g., 610a, 610b, ..., and 610h) of the ROI on the second region 620. The processor 310 determines that a display order of the list of the plurality of spectral Doppler images is 620a, 620b, ..., and 620h based on a peak velocity of blood flow. The display 320 displays the plurality of spectral Doppler images of the list in the determined display order on the second region 620. In detail, the plurality of spectral Doppler images 620a, 620b, ..., 620h are displayed in a magnitude order of peak velocity values of blood flow on the second region 620 from the top to the bottom of the second region 620.

According to the present embodiment, the user may grasp at once a blood flow distribution in the ROI by using a plurality of spectral Doppler images displayed in a magnitude order of peak velocity values of blood flow, and may easily find a spectral Doppler image corresponding to a portion desired to be diagnosed (e.g., a portion with cardiac regurgitation or renal blood vessels).

According to an embodiment, the plurality of sections of the first region 610 of the display 320 and the plurality of spectral Doppler images of the second region 620 of the display 320 may be mapped to indicators on the display 320. For example, referring to FIG. 6A, the spectral Doppler image 620a corresponding to the section 610a is mapped to an indicator 2 on the display 320. Also, the spectral Doppler image 620g corresponding to the section 610g is mapped to an indicator 7 on the display 320. According to an embodiment, each indicator of the ROI may be set based on a user input.

According to an embodiment, the processor 310 may set a color corresponding to each of the plurality of sections of the ROI. The display 320 may apply the color set by the processor 310 to at least one of the plurality of sections of the ROI 520 of the ultrasound image 500 displayed on the first region 610 and at least one of the plurality of spectral Doppler images displayed on the second region 620. According to an embodiment, the color corresponding to each section may be displayed on the entire section. According to another embodiment, the color corresponding to each section may be displayed on a part of the section.

For example, referring to FIG. 6A, the display 320 displays the ultrasound image and the plurality of spectral Doppler images by applying colors, which are set by the processor 310 to 8 sections 610a, 610b, ..., 610h of the ROI 520, to the plurality of sections of the first region 610 of the display 320 and the plurality of spectral Doppler images 620a, 620b, ...,620h of the second region 620 of the display 320. In detail, the processor 310 sets a color corresponding to the section 610a from among the plurality of sections to a yellow color, and the display 320 displays the section 610a and the spectral Doppler image 620a corresponding to the section 610a by applying the yellow color to the section 610a and the spectral Doppler image 620a corresponding to the section 610a.

According to an embodiment, the processor 310 may set that colors respectively corresponding to the plurality of sections of the ROI are different from one another. For example, referring to FIG. 6A, the processor 310 may set colors of the plurality of sections 610a, 610b, 610c, and 610d to yellow, green, sky blue, and blue colors that are different from one another, and the display 320 may apply the set colors to the sections 610a, 610b, 610c, and 610d and the plurality of spectral Doppler images 620a, 620b, 620c, and 620d respectively corresponding to the sections 610a, 610b, 610c, and 610d.

The user may recognize at once a plurality of sections mapped to corresponding colors and displayed on the display 320 and a plurality of spectral Doppler images respectively corresponding to the plurality of sections. Accordingly, the user may easily grasp which section's blood flow of the object is related to each spectral Doppler image.

FIG. 6B is a view illustrating an example where only spectral Doppler images whose peak velocity values of blood flow from among a plurality of spectral Doppler images displayed on the second region 620 of the display 320 of FIG. 6A are greater than a preset value are displayed on the second region 620 of the display 320.

Referring to FIG. 6B, the display 320 displays only a plurality of spectral Doppler images (e.g., 620a, 620b, 620c, and 620d) corresponding to 4 sections 610a, 610b, 610c, and 610d from among 8 sections of the ROI whose peak velocity values of blood flow are greater than a preset value. The processor 310 determines that a display order of a list of a plurality of spectral Doppler images is 620a, 620b, ..., and 620h based on a peak velocity of blood flow. The display 320 displays a plurality of spectral Doppler images of the list in the determined display order on the second region 620. In detail, the plurality of spectral Doppler images 620a, 620b, 620c, and 620d whose peak velocity values of blood flow are greater than a preset value are displayed in a magnitude order of peak velocity values of blood value from 620a to 620d on the second region 620 from the top to the bottom of the second region 620.

Accordingly, the user may easily recognize spectral Doppler images having peak velocity values of blood flow equal to or greater than a predetermined value. Also, the user may easily recognize spectral Doppler images of portions where blood vessels exist in the ROI of the object.

According to an embodiment, the display order of the plurality of spectral Doppler images displayed on the second region 620 of the display 320 may be updated in every preset cycle. Referring to FIGS. 6A and 6B, the magnitude order of peak velocity values of blood flow of the plurality of spectral Doppler images respectively corresponding to the 8 sections may be changed after the movement of the object.

For example, the spectral Doppler image 620g corresponding to the section 610g from among the plurality of spectral Doppler images may have a highest peak velocity value of blood flow after the movement of the object. The processor 310 may update the display order of the plurality of spectral Doppler images based on a change in magnitudes of peak velocity values of blood flow of the plurality of spectral Doppler images in every preset cycle. The display 320 may display, on the second region 620, in the updated display order, the plurality of spectral Doppler images of the list by reflecting the movement of the object.

According to another embodiment, the processor 310 may update the display order whenever the magnitude order of peak velocity values of blood flow is changed.

As the display order is updated, the user may determine which section has a highest peak velocity value of blood flow in the ROI of the object in real time, irrespective of the movement of the object.

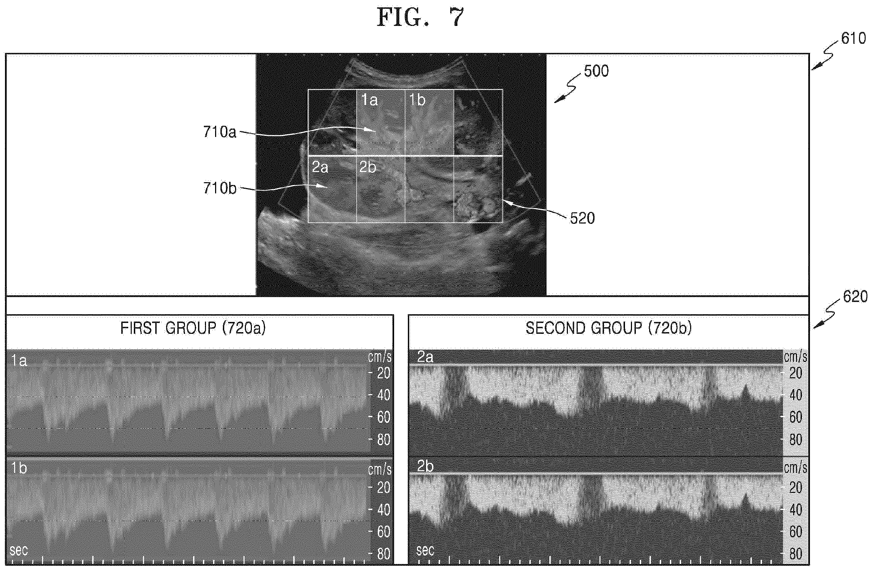

FIG. 7 is a view illustrating an example where the ultrasound imaging apparatus 300 displays the ultrasound image 500 of an object on the first region 610 of the display 320, and displays, on the second region 620, a plurality of spectral Doppler images according to groups having the same cycle, the same phase, and the same pattern of blood flow, according to an embodiment.

According to an embodiment, the processor 310 may classify a plurality of spectral Doppler images based on blood flow data into groups having the same cycle, the same phase, and the same pattern of blood flow. Spectral Doppler images respectively corresponding to sections including the same blood vessel in an ROI may have the same cycle, the same phase, and the same pattern of blood flow. Accordingly, the ultrasound imaging apparatus 300 may separately provide a plurality of spectral Doppler images of different blood vessels in the ROI to a user by classifying the plurality of spectral Doppler images into groups having the same cycle, the same phase, and the same pattern of blood flow.

For example, referring to FIG. 7, the processor 310 may classify a plurality of spectral Doppler images into a first group 720a and a second group 720b according to whether the plurality of spectral Doppler images have the same cycle, the same phase, and the same pattern of blood flow based on blood flow data. The display 320 displays the plurality of spectral Doppler images of the first group 720a and the second group 720b generated by the processor 310 on different portions of the second region 620 in a display order determined by the processor 310 based on a peak velocity value of blood flow. The object is a kidney and a plurality of sections 710a which corresponds to the first group 720a and a plurality of sections 710b which corresponds to the second group 720b include different blood vessels of the kidney.

According to another embodiment, even when a position where cardiac regurgitation occurs is periodically changed, the user may diagnose the cardiac regurgitation by easily finding the position where the cardiac regurgitation occurs. In detail, the ultrasound imaging apparatus 300 may set the heart as an ROI and may divide the set ROI into a plurality of sections. The ultrasound imaging apparatus 300 may display spectral Doppler images respectively corresponding to the plurality of sections based on a velocity of blood flow. The ultrasound imaging apparatus 300 may classify the plurality of spectral Doppler images into a plurality of groups according to whether the plurality of spectral Doppler images have the same cycle, the same phase, and the same pattern of blood flow based on blood flow data. The user may determine a spectral Doppler image of a position where cardiac regurgitation occurs based on phases and patterns of spectral Doppler images of each of the groups.

According to an embodiment, the processor 310 may set a color corresponding to each of the groups of the plurality of spectral Doppler images classified based on the blood flow data. The display 320 may apply the color, which is set according to each of the groups, to a plurality of sections of the first region of the display 320 and spectral Doppler images of the second region of the display 320.

For example, referring to FIG. 7, the processor 310 sets colors corresponding to the first group 720a and the second group 720b to yellow and sky blue, and the display 320 displays a plurality of sections 710a and 710b of the first region 610 and the groups 720a and 720b of spectral Doppler images of the second region 620 by applying the set colors to the plurality of sections 710a and 710b of the first region 610 and the groups 720a and 720b of spectral Doppler images of the second region 620.

According to the present embodiment, the user may easily grasp which blood vessel of the object is related to spectral Doppler images included in each of groups by observing a plurality of sections and a plurality of spectral Doppler images mapped to corresponding colors according to the groups on the display 320.

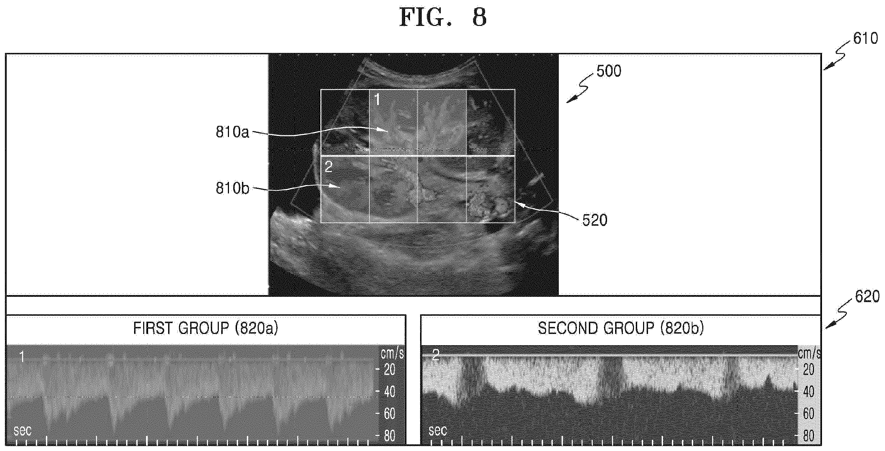

FIG. 8 is a view illustrating an example where the ultrasound imaging apparatus 300 displays an ultrasound image of an object on the first region 610 of the display 320, and displays a plurality of spectral Doppler images synthesized according to groups having the same cycle and the same pattern of blood flow on the second region 620, according to an embodiment.

According to an embodiment, the processor 310 may classify a plurality of spectral Doppler images into groups having the same cycle, the same phase, and the same pattern of blood flow. The processor 310 may synthesize a plurality of spectral Doppler images included in each of the groups into one spectral Doppler image having a continuous spectrum on the same time axis. The synthesizing of the spectral Doppler images may be performed by using, for example, signal combination or convolution. The display 320 may display the synthesized spectral Doppler images according to the groups on different portions of the second region 620.

For example, referring to FIG. 8, the processor 310 synthesizes a plurality of spectral Doppler images included in each of the first group 720a and the second group 720b into one spectral Doppler image having a continuous spectrum on the same time axis. The display 320 displays the synthesized spectral Doppler images on different portions of the second region 620 according to the groups 820a and 820b.

According to an embodiment, the processor 310 may set a color corresponding to each of the groups of the synthesized spectral Doppler images. The display 320 may apply the color, which is set according to each of the groups, to a plurality of sections of the first region 610 of the display 320 and spectral Doppler images of the second region 620 of the display 320.

For example, referring to FIG. 8, the processor 310 sets colors corresponding to the first group 820a and the second group 820b to yellow and sky blue, and the display 320 displays a plurality of sections 810a and 810b of the first region 610 and groups 820a and 820b of spectral Doppler images of the second region 620 by applying the set colors to the plurality of sections 810a and 810b of the first region 610 and the groups 820a and 820b of spectral Doppler images of the second region 620.

It is found that two spectral Doppler images each having a discontinuous spectrum on a time axis in the first group 710a are synthesized into one spectral Doppler image having a continuous spectrum on the same time axis.

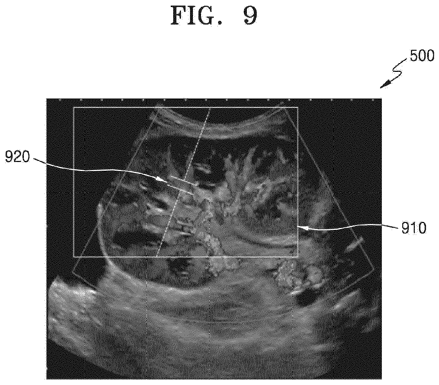

FIG. 9 is a view illustrating an example where the ultrasound imaging apparatus 300 tracks a section corresponding to a spectral Doppler images having a highest peak velocity value of blood flow from among a plurality of spectral Doppler images, according to an embodiment.

According to an embodiment, the processor 310 may determine a spectral Doppler image having a highest peak velocity value of blood flow from among a plurality of spectral Doppler images respectively corresponding to a plurality of sections of a preset ROI. The processor 310 may set a section corresponding to the spectral Doppler image having the highest peak velocity value of blood flow from among the plurality of sections as a tracking section. The processor 310 may track the set tracking section in every preset cycle, and may re-set the ROI in every preset cycle so that the tracking section is located at the center of the ROI.

For example, referring to FIGS. 6A and 9, a spectral Doppler image having a highest peak velocity value of blood flow from among a plurality of spectral Doppler images respectively corresponding to a plurality of sections in FIG. 6A is 620a. A section corresponding to the spectral Doppler image 620a is 610a. The processor 310 sets the section 610a as a tracking section. The processor 310 tracks the section 610a in every preset cycle, and re-sets the ROI in every preset cycle so that the section 610a is located at the center of the ROI. FIG. 9 illustrates an ROI 910 re-set so that the section 610a of FIG. 6A is located at the center of the re-set ROI 910. In this case, a sample gate 920 may be located at the center of the re-set ROI 910.

According to an embodiment, the processor 310 may re-set a size of the ROI when re-setting the ROI so that a tracking section is located at the center. The re-setting of the size of the ROI may be performed based on a user's input. For example, the ultrasound imaging apparatus 300 may receive an input that reduces a size of the re-set ROI to a size of a tracking section, and the processor 310 may reduce the size of the ROI to the size of the tracking section based on the input that reduces the size of the ROI.

According to an embodiment, the ultrasound imaging apparatus 300 may perform the method including operations 420 through 450 of FIG. 4 based on the re-set ROI.

Embodiments may be implemented on computer-readable recording media storing instructions and data executable by computers. The instructions may be stored as program codes, and when being executed by a processor, may cause a predetermined program module to be generated and a predetermined operation to be performed. Also, when executed by the processor, the instructions may cause predetermined operations of the disclosed embodiments to be performed.

## Claims

1. An ultrasound imaging apparatus (300) comprising:
at least one processor (310) configured to set a region of interest (ROI) (520) in an ultrasound image (500) of an object, divide the ROI (520) into a plurality of sections (610a, 610b, ...), generate a plurality of spectral Doppler images (620a, 620b, ...) respectively corresponding to the plurality of sections (610a, 610b, ...), and determine, based on a peak velocity value of blood flow, a display order of a list of the plurality of spectral Doppler images (620a, 620b, ...); and
a display (320) configured to display the ultrasound image (500) of the object on a first region (610) of the display and display, on a second region (620) of the display, in the determined display order the spectral Doppler images (620a, 620b, ...) whose peak velocity values of blood flow are greater than a preset value from among the plurality of spectral Doppler images (620a, 620b, ...) of the list; the display being further configured to map corresponding indicators between the sections (610a, 610b, ...) in the first region (610) of the display and the spectral Doppler images (620a, 620b, ...) in the second region (620) of the display.

2. The ultrasound imaging apparatus (300) of claim 1, wherein the at least one processor (310) is further configured to divide the set ROI (520) into the plurality of sections (610a, 610b, ...) based on a user input for at least one or a combination of a number of the plurality of sections, a size of each of the plurality of sections, and a shape of each of the plurality of sections.

3. The ultrasound imaging apparatus (300) of claim 1 or 2, wherein the at least one processor (310) is further configured to divide the set ROI (520) into the plurality of sections (610a, 610b, ...) by automatically recognizing an anatomical structure in the set ROI and automatically determining, based on the recognized anatomical structure, at least one or a combination of a number of the plurality of sections, a size of each of the plurality of sections, and a shape of each of the plurality of sections.

4. The ultrasound imaging apparatus (300) of claim 1, 2 or 3, wherein the at least one processor (310) is further configured to update the determined display order in each of preset cycles.

5. The ultrasound imaging apparatus (300) of any of claims 1-4, wherein the at least one processor (310) is further configured to set a color corresponding to at least one of the plurality of sections (610a, 610b, ...), and
the display (320) is further configured to display at least one of the plurality of sections of the first region and at least one of the plurality of spectral Doppler images (620a, 620b, ...) of the second region by applying the set color to the at least one of the plurality of sections of the first region and the at least one of the plurality of spectral Doppler images of the second region.

6. The ultrasound imaging apparatus (300) of any of claims 1-5, wherein the at least one processor (310) is further configured to classify the plurality of spectral Doppler images (620a, 620b, ...) into groups having the same cycle, the same phase, and the same pattern of blood flow, based on blood flow data, and
the display (320) is further configured to display the plurality of spectral Doppler images according to the groups on different portions of the second region in the display order.

7. The ultrasound imaging apparatus (300) of claim 6, wherein the at least one processor (310) is further configured to set a color corresponding to each of the classified groups, and
the display (320) is further configured to apply the set color to at least one of the plurality of sections of the first region and at least one of the plurality of spectral Doppler images (620a, 620b, ...) of the second region.

8. The ultrasound imaging apparaus (300) of claim 6 or 7, wherein, with respect to the plurality of spectral Doppler images (620a, 620b, ...) classified into the groups, the at least one processor is further configured to synthesize, for each group, spectral Doppler images included in a group into one spectral Doppler image having a continuous spectrum on the same time axis, and
the display (320) is further configured to display the synthesized spectral Doppler images on different portions of the second region according to the groups.

9. The ultrasound imaging apparatus (300) of claim 8, wherein the at least one processor (310) is further configured to set a color corresponding to each of the classified groups, and
the display (320) is further configured to apply the set color to at least one of the plurality of sections of the first region and at least one of the plurality of spectral Doppler images (620a, 620b, ...) of the second region.

10. The ultrasound imaging apparatus (300) of any of claims 1-9, wherein the at least one processor (310) is further configured to re-set the ROI (520) so that a section corresponding to a spectral Doppler image having a highest peak velocity value of blood flow from among the plurality of spectral Doppler images (620a, 620b, ...) is located at the center of the ROI.

11. A method of controlling an ultrasound imaging apparatus (300), the method comprising:
setting a region of interest (ROI) (520) in an ultrasound image (500) of an object;
dividing the ROI (520) into a plurality of sections (610a, 610b, ...); generating a plurality of spectral Doppler images (620a, 620b, ...) respectively corresponding to the plurality of sections (610a, 610b, ...);
determining a display order of a list of the plurality of spectral Doppler images (620a, 620b, ...), based on a peak velocity value of blood flow;
displaying the ultrasound image (500) of the object on a first region (610) of a display (320) and displaying, on a second region (620) of the display (320) in the determined display order the spectral Doppler (620a, 620b, ...) whose peak velocity values of blood flow are greater than a preset value from among the plurality of spectral Doppler (620a, 620b, ...) of the list; and
displaying corresponding indicators between the sections (610a, 610b, ...) in the first region (610) of the display and the spectral Doppler images (620a, 620b, ...) in the second region (620) of the display.

12. The method of claim 11, further comprising:
setting a color corresponding to at least one of the plurality of sections (610a, 610b, ...); and
displaying at least one of the plurality of sections of the first region and at least one of the plurality of spectral Doppler images (620a, 620b, ...) of the second region by applying the set color to the at least one of the plurality of sections of the first region and the at least one of the plurality of spectral Doppler images of the second region.

13. The method of claim 11 or 12, further comprising:
classifying the plurality of spectral Doppler images (620a, 620b, ...), based on blood flow data, into groups having the same cycle, the same phase, and the same pattern of blood flow; and
displaying the plurality of spectral Doppler images according to the groups on different portions of the second region in the display order.

14. The method of claim 13, further comprising:
with respect to the plurality of spectral Doppler images (620a, 620b, ...) classified into the groups, synthesizing, for each group, spectral Doppler images included therein into one spectral Doppler image having a continuous spectrum on the same time axis; and
displaying the synthesized spectral Doppler images on different portions of the second region according to the groups.

15. A computer program product comprising a computer-readable storage medium, the computer-readable storage medium storing instructions for performing the method steps of any of claims 11-14.

## Patentansprüche

1. Ultraschallbildgebungsvorrichtung (300), die Folgendes umfasst:
mindestens einen Prozessor (310), der dazu konfiguriert ist, einen Bereich von Interesse (ROI, Region Of Interest) (520) in einem Ultraschallbild (500) eines Objekts einzustellen, den ROI (520) in eine Vielzahl von Abschnitten (610a, 610b, ...) zu unterteilen, eine Vielzahl von spektralen Dopplerbildern (620a, 620b, ...) zu erzeugen, die jeweils der Vielzahl von Abschnitten (610a, 610b, ...) entsprechen, und basierend auf einem Spitzengeschwindigkeitswert des Blutflusses eine Anzeigereihenfolge einer Liste der Vielzahl von spektralen Dopplerbildern (620a, 620b, ...) zu bestimmen; und
eine Anzeige (320), die dazu konfiguriert ist, das Ultraschallbild (500) des Objekts auf einem ersten Bereich (610) der Anzeige anzuzeigen und die spektralen Dopplerbilder (620a, 620b, ...) aus der Vielzahl von spektralen Dopplerbildern (620a, 620b, ...) der Liste, deren Spitzengeschwindigkeitswerte des Blutflusses größer als ein voreingestellter Wert sind, auf einem zweiten Bereich (620) der Anzeige in der bestimmten Anzeigereihenfolge anzuzeigen;
wobei die Anzeige ferner dazu konfiguriert ist, entsprechende Indikatoren zwischen den Abschnitten (610a, 610b, ...) in dem ersten Bereich (610) der Anzeige und den spektralen Dopplerbildern (620a, 620b, ...) in dem zweiten Bereich (620) der Anzeige abzubilden.

2. Ultraschallbildgebungsvorrichtung (300) nach Anspruch 1, wobei der mindestens eine Prozessor (310) ferner dazu konfiguriert ist, den eingestellten ROI (520) in die Vielzahl von Abschnitten (610a, 610b, ...) zu unterteilen, und zwar basierend auf einer Benutzereingabe für mindestens einen oder eine Kombination aus einer Anzahl der Vielzahl von Abschnitten, einer Größe von jedem aus der Vielzahl von Abschnitten und einer Form von jedem aus der Vielzahl von Abschnitten.

3. Ultraschallbildgebungsvorrichtung (300) nach Anspruch 1 oder 2, wobei der mindestens eine Prozessor (310) ferner dazu konfiguriert ist, den eingestellten ROI (520) in die Vielzahl von Abschnitten (610a, 610b, ...) zu unterteilen durch automatisches Erkennen einer anatomischen Struktur in dem eingestellten ROI und automatisches Bestimmen, basierend auf der erkannten anatomischen Struktur, mindestens eines oder einer Kombination aus einer Anzahl der Vielzahl von Abschnitten, einer Größe von jedem aus der Vielzahl von Abschnitten und einer Form von jedem aus der Vielzahl von Abschnitten.

4. Ultraschallbildgebungsvorrichtung (300) nach Anspruch 1, 2 oder 3, wobei der mindestens eine Prozessor (310) ferner dazu konfiguriert ist, die bestimmte Anzeigereihenfolge in jedem Zyklus unter voreingestellten Zyklen zu aktualisieren.

5. Ultraschallbildgebungsvorrichtung (300) nach einem der Ansprüche 1 bis 4, wobei der mindestens eine Prozessor (310) ferner dazu konfiguriert ist, eine Farbe einzustellen, die mindestens einem aus der Vielzahl von Abschnitten (610a, 610b, ...) entspricht, und
die Anzeige (320) ferner dazu konfiguriert ist, mindestens einen aus der Vielzahl von Abschnitten des ersten Bereichs und mindestens eines der Vielzahl von spektralen Dopplerbildern (620a, 620b, ...) des zweiten Bereichs anzuzeigen durch Anwenden der eingestellten Farbe auf den mindestens einen aus der Vielzahl von Abschnitten des ersten Bereichs und das mindestens eine aus der Vielzahl von spektralen Dopplerbildern des zweiten Bereichs.

6. Ultraschallbildgebungsvorrichtung (300) nach einem der Ansprüche 1 bis 5, wobei der mindestens eine Prozessor (310) ferner dazu konfiguriert ist, die Vielzahl von spektralen Dopplerbildern (620a, 620b, ...) in Gruppen zu klassifizieren, die den gleichen Zyklus, die gleiche Phase und das gleiche Blutflussmuster aufweisen, und zwar basierend auf Blutflussdaten, und
die Anzeige (320) ferner dazu konfiguriert ist, die Vielzahl von spektralen Dopplerbildern gemäß den Gruppen auf unterschiedlichen Teilbereichen des zweiten Bereichs in der Anzeigereihenfolge anzuzeigen.

7. Ultraschallbildgebungsvorrichtung (300) nach Anspruch 6, wobei der mindestens eine Prozessor (310) ferner dazu konfiguriert ist, eine Farbe einzustellen, die jeder der klassifizierten Gruppen entspricht, und
die Anzeige (320) ferner dazu konfiguriert ist, die eingestellte Farbe auf mindestens einen aus der Vielzahl von Abschnitten des ersten Bereichs und mindestens eines aus der Vielzahl von spektralen Dopplerbildern (620a, 620b, ...) des zweiten Bereichs anzuwenden.

8. Ultraschallbildgebungsvorrichtung (300) nach Anspruch 6 oder 7, wobei in Bezug auf die Vielzahl von spektralen Dopplerbildern (620a, 620b, ...), die in die Gruppen klassifiziert sind, der mindestens eine Prozessor ferner dazu konfiguriert ist, für jede Gruppe spektrale Dopplerbilder, die in einer Gruppe enthalten sind, zu einem spektralen Dopplerbild mit einem kontinuierlichen Spektrum auf derselben Zeitachse zu synthetisieren, und
die Anzeige (320) ferner dazu konfiguriert ist, die synthetisierten spektralen Dopplerbilder gemäß den Gruppen auf verschiedenen Teilbereichen des zweiten Bereichs anzuzeigen.

9. Ultraschallbildgebungsvorrichtung (300) nach Anspruch 8, wobei der mindestens eine Prozessor (310) ferner dazu konfiguriert ist, eine Farbe einzustellen, die jeder der klassifizierten Gruppen entspricht, und
die Anzeige (320) ferner dazu konfiguriert ist, die eingestellte Farbe auf mindestens einen aus der Vielzahl von Abschnitten des ersten Bereichs und mindestens eines aus der Vielzahl von spektralen Dopplerbildern (620a, 620b, ...) des zweiten Bereichs anzuwenden.

10. Ultraschallbildgebungsvorrichtung (300) nach einem der Ansprüche 1 bis 9, wobei der mindestens eine Prozessor (310) ferner dazu konfiguriert ist, den ROI (520) neu einzustellen, so dass ein Abschnitt, der einem spektralen Dopplerbild aus der Vielzahl von spektralen Dopplerbildern (620a, 620b, ...) entspricht, das einen höchsten Spitzengeschwindigkeitswert des Blutflusses aufweist, sich in der Mitte des ROI befindet.

11. Verfahren zur Steuerung einer Ultraschallbildgebungsvorrichtung (300), wobei das Verfahren Folgendes umfasst:
Einstellen eines Bereichs von Interesse (ROI, Region Of Interest) (520) in einem Ultraschallbild (500) eines Objekts;
Unterteilen des ROI (520) in eine Vielzahl von Abschnitten (610a, 610b, ...);
Erzeugen einer Vielzahl von spektralen Dopplerbildern (620a, 620b, ...), die jeweils der Vielzahl von Abschnitten (610a, 610b, ...) entsprechen;
Bestimmen einer Anzeigereihenfolge einer Liste aus der Vielzahl von spektralen Dopplerbildern (620a, 620b, ...) basierend auf einem Spitzengeschwindigkeitswert des Blutflusses;
Anzeigen des Ultraschallbilds (500) des Objekts auf einem ersten Bereich (610) einer Anzeige (320) und Anzeigen der spektralen Dopplerbilder (620a, 620b, ...) aus der Vielzahl von spektralen Dopplerbildern (620a, 620b, ...) der Liste, deren Spitzengeschwindigkeitswerte des Blutflusses größer als ein voreingestellter Wert sind, auf einem zweiten Bereich (620) der Anzeige in der bestimmten Anzeigereihenfolge; und
Anzeigen entsprechender Indikatoren zwischen den Abschnitten (610a, 610b, ...) in dem ersten Bereich (610) der Anzeige und den spektralen Dopplerbildern (620a, 620b, ...) in dem zweiten Bereich (620) der Anzeige.

12. Verfahren nach Anspruch 11, das ferner Folgendes umfasst:
Einstellen einer Farbe, die mindestens einem aus der Vielzahl von Abschnitten (610a, 610b, ...) entspricht; und
Anzeigen mindestens eines aus der Vielzahl von Abschnitten des ersten Bereichs und mindestens eines aus der Vielzahl von spektralen Dopplerbildern (620a, 620b, ...) des zweiten Bereichs durch Anwenden der eingestellten Farbe auf den mindestens einen aus der Vielzahl von Abschnitten des ersten Bereichs und das mindestens eine aus der Vielzahl von spektralen Dopplerbildern des zweiten Bereichs.

13. Verfahren nach Anspruch 11 oder 12, das ferner Folgendes umfasst:
Klassifizieren der Vielzahl von spektralen Dopplerbildern (620a, 620b, ...) in Gruppen, die den gleichen Zyklus, die gleiche Phase und das gleiche Blutflussmuster aufweisen, und zwar basierend auf Blutflussdaten; und
Anzeigen der Vielzahl von spektralen Dopplerbildern gemäß den Gruppen auf unterschiedlichen Teilbereichen des zweiten Bereichs in der Anzeigereihenfolge.

14. Verfahren nach Anspruch 13, das ferner Folgendes umfasst:
in Bezug auf die Vielzahl von spektralen Dopplerbildern (620a, 620b, ...), die in die Gruppen klassifiziert sind, Synthetisieren spektraler Dopplerbilder, die in einer Gruppe enthalten sind, zu einem spektralen Dopplerbild mit einem kontinuierlichen Spektrum auf derselben Zeitachse, und zwar für jede Gruppe; und
Anzeigen der synthetisierten spektralen Dopplerbilder gemäß den Gruppen auf verschiedenen Teilbereichen des zweiten Bereichs.

15. Computerprogrammprodukt mit einem computerlesbaren Speichermedium, wobei in dem computerlesbaren Speichermedium Anweisungen zur Durchführung der Verfahrensschritte nach einem der Ansprüche 11 bis 14 gespeichert sind.

## Revendications

1. Appareil échographique (300) comprenant :
au moins un processeur (310) conçu pour définir une région d'intérêt (ROI, region of interest) (520) dans une image échographique (500) d'un objet, diviser la ROI (520) en une pluralité de sections (610a, 610b, ...), générer une pluralité d'images Doppler spectrales (620a, 620b, ...) correspondant respectivement à la pluralité de sections (610a, 610b, ...), et déterminer, compte tenu d'une valeur de vitesse maximale de circulation sanguine, l'ordre d'affichage d'une liste de la pluralité d'images Doppler spectrales (620a, 620b, ...), et
un écran (320) conçu pour afficher l'image échographique (500) de l'objet sur une première région (610) de l'écran et pour afficher, sur une deuxième région (620) de l'écran, dans l'ordre d'affichage déterminé, les images Doppler spectrales (620a, 620b, ...) dont les valeurs de vitesse maximale de circulation sanguine sont supérieures à une valeur prédéfinie parmi la pluralité d'images Doppler spectrales (620a, 620b, ...) de la liste ;
l'écran étant conçu en outre pour effectuer la mise en correspondance d'indicateurs correspondants entre les sections (610a, 610b, ...) de la première région (610) de l'écran et les images Doppler spectrales (620a, 620b, ...) de la deuxième région (620) de l'écran.

2. Appareil échographique (300) selon la revendication 1, dans lequel l'au moins un processeur (310) est conçu en outre pour diviser la ROI (520) définie en ladite pluralité de sections (610a, 610b, ...) compte tenu d'une entrée utilisateur concernant au moins un paramètre ou une combinaison des paramètres suivants : le nombre de la pluralité de sections, la taille de chacune des sections de la pluralité de sections et la forme de chacune des sections de la pluralité de sections.

3. Appareil échographique (300) selon la revendication 1 ou 2, dans lequel l'au moins un processeur (310) est conçu en outre pour diviser la ROI (520) définie en ladite pluralité de sections (610a, 610b, ...) par reconnaissance automatique d'une structure anatomique dans la ROI définie et détermination automatique, compte tenu de la structure anatomique reconnue, d'au moins un paramètre ou d'une combinaison des paramètres suivants : le nombre de la pluralité de sections, la taille de chacune des sections de la pluralité de sections et la forme de chacune des sections de la pluralité de sections.

4. Appareil échographique (300) selon la revendication 1, 2 ou 3, dans lequel l'au moins un processeur (310) est conçu en outre pour actualiser l'ordre d'affichage déterminé dans chaque cycle parmi des cycles prédéfinis.

5. Appareil échographique (300) selon l'une quelconque des revendications 1 à 4, dans lequel l'au moins un processeur (310) est conçu en outre pour définir une couleur correspondant à au moins une des sections de la pluralité de sections (610a, 610b, ...), et
l'écran (320) est conçu en outre pour afficher au moins une section de la pluralité de sections de la première région et au moins une image de la pluralité d'images Doppler spectrales (620a, 620b, ...) de la deuxième région par application de la couleur définie à l'au moins une section de la pluralité de sections de la première région et à l'au moins une image de la pluralité d'images Doppler spectrales de la deuxième région.

6. Appareil échographique (300) selon l'une quelconque des revendications 1 à 5, dans lequel l'au moins un processeur (310) est conçu en outre pour classer la pluralité d'images Doppler spectrales (620a, 620b, ...) en des groupes ayant le même cycle, la même phase et la même configuration de circulation sanguine, compte tenu de données relatives à la circulation sanguine, et
l'écran (320) est conçu en outre pour afficher la pluralité d'images Doppler spectrales conformément auxdits groupes sur différentes parties de la deuxième région dans l'ordre d'affichage.

7. Appareil échographique (300) selon la revendication 6, dans lequel l'au moins un processeur (310) est conçu en outre pour définir une couleur correspondant à chacun des groupes classés, et l'écran (320) est conçu en outre pour appliquer la couleur définie à au moins une section de la pluralité de sections de la première région et à au moins une image de la pluralité d'images Doppler spectrales (620a, 620b, ...) de la deuxième région.

8. Appareil échographique (300) selon la revendication 6 ou 7, dans lequel, eu égard à la pluralité d'images Doppler spectrales (620a, 620b, ...) classées en lesdits groupes, l'au moins un processeur est conçu en outre pour synthétiser, pour chaque groupe, les images Doppler spectrales présentes dans un groupe en une même image Doppler spectrale présentant un spectre continu sur le même axe temporel, et
l'écran (320) est conçu en outre pour afficher les images Doppler spectrales synthétisées sur différentes parties de la deuxième région conformément aux groupes.

9. Appareil échographique (300) selon la revendication 8, dans lequel l'au moins un processeur (310) est conçu en outre pour définir une couleur correspondant à chacun des groupes classés, et l'écran (320) est conçu en outre pour appliquer la couleur définie à au moins une section de la pluralité de sections de la première région et à au moins une image de la pluralité d'images Doppler spectrales (620a, 620b, ...) de la deuxième région.

10. Appareil échographique (300) selon l'une quelconque des revendications 1 à 9, dans lequel l'au moins un processeur (310) est conçu en outre pour redéfinir la ROI (520) de façon qu'une section correspondant à une image Doppler spectrale présentant la plus haute valeur de vitesse maximale de circulation sanguine parmi la pluralité d'images Doppler spectrales (620a, 620b, ...) soit située au centre de la ROI.

11. Procédé de commande d'un appareil échographique (300), le procédé comprenant :
la définition d'une région d'intérêt (ROI, region of interest) (520) dans une image échographique (500) d'un objet,
la division de la ROI (520) en une pluralité de sections (610a, 610b, ...),
la génération d'une pluralité d'images Doppler spectrales (620a, 620b, ...) correspondant respectivement à la pluralité de sections (610a, 610b, ...),
la détermination de l'ordre d'affichage d'une liste de la pluralité d'images Doppler spectrales (620a, 620b, ...), compte tenu d'une valeur de vitesse maximale de circulation sanguine,
l'affichage de l'image échographique (500) de l'objet sur une première région (610) d'un écran (320), et l'affichage, sur une deuxième région (620) de l'écran (320), dans l'ordre d'affichage déterminé, des images Doppler spectrales (620a, 620b, ...) dont les valeurs de vitesse maximale de circulation sanguine sont supérieures à une valeur prédéfinie parmi la pluralité d'images Doppler spectrales (620a, 620b, ...) de la liste, et
l'affichage d'indicateurs correspondants entre les sections (610a, 610b, ...) de la première région (610) de l'écran et les images Doppler spectrales (620a, 620b, ...) de la deuxième région (620) de l'écran.

12. Procédé selon la revendication 11, comprenant en outre :
la définition d'une couleur correspondant à au moins une section de la pluralité de sections (610a, 610b, ...), et
l'affichage d'au moins une section de la pluralité de sections de la première région et d'au moins une image de la pluralité d'images Doppler spectrales (620a, 620b, ...) de la deuxième région par application de la couleur définie à l'au moins une section de la pluralité de sections de la première région et à l'au moins une image de la pluralité d'images Doppler spectrales de la deuxième région.

13. Procédé selon la revendication 11 ou 12, comprenant en outre :
le classement de la pluralité d'images Doppler spectrales (620a, 620b, ...), compte tenu de données relatives à la circulation sanguine, en des groupes ayant le même cycle, la même phase et la même configuration de circulation sanguine, et
l'affichage de la pluralité d'images Doppler spectrales conformément auxdits groupes sur différentes parties de la deuxième région dans l'ordre d'affichage.

14. Procédé selon la revendication 13, comprenant en outre :
eu égard à la pluralité d'images Doppler spectrales (620a, 620b, ...) classées en lesdits groupes, la synthétisation, pour chaque groupe, d'images Doppler spectrales qui y sont présentes en une même image Doppler spectrale présentant un spectre continu sur le même axe temporel, et
l'affichage des images Doppler spectrales synthétisées sur différentes parties de la deuxième région conformément aux groupes.

15. Produit-programme d'ordinateur comprenant un support d'enregistrement lisible par ordinateur, le support d'enregistrement lisible par ordinateur contenant des instructions permettant de réaliser les étapes de procédé selon l'une quelconque des revendications 11 à 14.
